# EUROPÄISCHE PATENTSCHRIFT

(11) **EP 1 609 466 B1**
(45) Veröffentlichungstag und Bekanntmachung des Hinweises auf die Patenterteilung: **10.11.2010**
(21) Anmeldenummer: 05012880.0
(22) Anmeldetag: 15.06.2005
(51) Int. Cl.: A61K 31/085, A61K 31/12, A61K 31/192, A61K 31/05, A61P 3/00

(54) **Pharmazeutische Zusammensetzung, Verwendung dieser pharmazeutischen Zusammensetzung zur Herstellung eines Medikaments und/oder eines Nahrungsergänzungmittels zur Behandlung von Übergewicht und/oder Symptomen, die mit Übergewicht einhergehen, beim Menschen oder Säugetier, sowie Verfahren zur Herstellung dieser pharmazeutischen Zusammensetzung**
Pharmaceutical compositions and uses thereof in the preparation of a medicament and/or food compostion for the treatment of overweight and/or correlated symptoms in humans and mammalian as well as process of preparation
Composition pharmaceutique et sa utilisation pour la mise en oeuvre d'un medicament pour le traitement du surpoids et/ou des symptomes correles dans le corps humain et animal et son procede de production.

(30) Priorität: 15.06.2004 US 579630 P
(43) Veröffentlichungstag der Anmeldung: 28.12.2005
(73) Patentinhaber: Ninkov, Dusan, Dr., San Diego, California 92128 (US); Mitrovic, Zeljko, Belgrade (YU); Milicevic, Svetislav, Elgrade (YU)
(72) Erfinder: Ninkov, Dusan, Dr., San Diego, California 92128 (US); Mitrovic, Zeljko, Belgrade (YU); Milicevic, Svetislav, Elgrade (YU)
(74) Vertreter: Patentanwälte Thömen & Körner

(56) Entgegenhaltungen:
- FR-A- 2 576 212
- US-A- 5 273 754
- US-A1- 2003 054 015
- US-A1- 2003 147 979
- US-A1- 2004 052 922
- US-A1- 2004 071 799
- US-B1- 6 201 014

## Beschreibung

Die Erfindung betrifft eine pharmazeutische Zusammensetzung, eine Verwendung dieser pharmazeutischen Zusammensetzung zur Herstellung eines Medikaments und/oder eines Nahrungsergänzungsmittels zur Behandlung von Übergewicht und/oder Symptomen, die mit Übergewicht einhergehen, beim Menschen oder Säugetier, sowie ein Verfahren zur Herstellung dieser pharmazeutischen Zusammensetzung.

### STAND DER TECHNIK

US-Patentschriften:
6,723,730 20. April 2004, Bakthavatchalam et al.
6,518,315 11. Februar 2003. B.D. Roufogalis et al.
20020082670 27. Juni 2002, D.S. Utley et al.
6,214,831 B1 10. April 2001, Y. Yokoo et al.
5,273,754 28. Dezember 1993, Mann

### Andere Veröffentlichungen:

V.N. Dedov, V.H. Tran, C.C. Duke, M. Connor, M.J. Christie, S. Mandadi, B.D. Roufogalis, Gingerols: a novel class of vanilloid receptor (VR1) agonists. Br. J. Pharmacol. 137(6):793-8, 2002.
M. Falchi, F. Ferrara, B. Dib, C. Gharib; Intracerebroventricular capsaicin and food intake in the rat. Drugs Exptl. Clin. Res. 27(2) 61-67, 2001.
M.P.G.M. Lejeune [1]; E.M.R. Kovacs [1]; M.S. Westerterp-Plantenga. Effect of capsaicin on substrate oxidation and weight maintenance after modest bodyweight loss in human subjects. Brit. J. Nutr. 90:3, 651-660, 2003.
A. Martinet, K. Hosterman, Y. Schutz. Thermogenic effects of commercially available plant preparations aimed at treating human obesity. Phytomedicine 6(4):231-238, 1999.
B. Sherman. Jolly no more, new research is piecing together the answers to fat's ponderous puzzle. Acumen, II(1):66-67, 2003.
A. Szallasi, P.M. Blumberg. Vanilloid (Capsaicin) Receptors and Mechanisms, Pharmacol. Rev. 51(2):159-212, 1999.
B.H. Yang, Z.G. Piao, Y.-B. Kim, C.-H. Lee, J.K. Lee, K. Park, J.S. Kim, S.B. Oh. Activation of vanilloid receptor 1 (VR1) by eugenol. J. Dent. Res. 82(10):781-785, 2003. M. Yoshioka, S. St-Pierre, V. Drapeau, I. Dionne, E. Doucet, M. Suzuki, A. Tremblay. Effects of red pepper on appetite and energy intake. Br. J. Nutr. 82(2):115-23, 1999.

### GEBIET DER ERFINDUNG

Die vorliegende Erfindung betrifft ein Verfahren zum Blockieren der irreversiblen Inaktivierung von Vanilloiden, chemischen Bestandteilen von ätherischen Ölen, die eine Vanillyl-Einheit (4-Hydroxy-3-methoxybenzyl-Einheit) enthalten.

Ätherische Öle werden in ein pharmazeutisch unbedenkliches Verdünnungsmittel oder Trägerstoffgemisch eingearbeitet, das ihre Schärfe und Reaktivität vermindert, ihre Wirksamkeit schützt und eine anhaltende und sequenzielle Freisetzung von Vanilloiden über einen langen Zeitraum ermöglicht.

Das Verfahren besteht aus dem Einarbeiten von individuellen ätherischen Ölen in Olivenöl, gefolgt vom Einarbeiten von Tröpfchen aus Olivenöl/ätherischem Öl in unbehandelte pyrogene Kieselsäure. Das Verfahren kann auf therapeutisch wirksame Mengen ätherischer Öle angewendet werden, wie beispielsweise Zimtblatt, Ingwer, Kurkuma, Thymian und pikante Winteröle, die unter anderem Eugenol, Carvacrol, Gingerol und Curcumin enthalten.

Insbesondere betrifft die vorliegende Erfindung eine pharmazeutische Zusammensetzung in Tablettenform für Erwachsene und Jugendliche, die therapeutisch wirksame Mengen Zimtblattöl, Ingwerextrakt, -oleoresin oder -öl, Kurkumaextraktöl oder -oleoresin und Kombinationen dieser Öle mit Kakaoextrakt, Zitronensäure, ätherischen Zitrusölen und eine Proteinfraktion aus weißer Kidneybohne mit alpha-Amylase-Inhibitionswirkung umfasst.

Insbesondere betrifft die vorliegende Erfindung eine pharmazeutische Zusammensetzung in Sirupform, die therapeutisch wirksame Mengen Zimtblattöi, Kurkumaextraktöl oder -oleoresin und Kombinationen dieser Öle mit Kakaoextrakt, Zitronensäure, ätherischen Zitrusölen und eine Proteinfraktion aus weißer Kidneybohne mit alpha-Amylase-Inhibitionswirkung umfasst.

Die Tablette und der Sirup werden an Personen vor einer Mahlzeit verabreicht, um deren Hunger zu zügeln, den Fettstoffwechsel anzukurbeln, ihr Wärmeregulafionsgleichgewicht zu verschieben und die Zuckeraufnahme zu reduzieren, wobei gleichzeitig Kalorienreduzierung und Gewichtsverlust gefördert werden.

### AUFGABE UND BESCHREIBUNG DER ERFINDUNG

Die 2004 von den Centers for Disease Control and Prevention (CDC) der US-Regierung neu veröffentlichten Statistiken offenbaren, dass mehr ais 64 Prozent der Erwachsenen in den USA übergewichtig oder fettleibig sind. Von diesen sind 64.5 Prozent übergewichtig (BMI von 25 oder mehr) und 30,5 Prozent fettleibig (BMI von 30 oder mehr).

Das Halten eines gesunden Gewichts ist sowohl aus kosmetischen als auch medizinischen Gründen wichtig. Obermäßiges Körpergewicht erhöht das Risiko der Entwicklung von Diabetes, Bluthochdruck, hohem Cholesterinspiegel, Herzerkrankung, Schlaganfall, einigen Krebsarten, Arthritis und anderen chronischen Zuständen. Die Verbindung von Fettleibigkeit und Erkrankung ist offensichtlicher, wenn man die Anteile von Eiwachsenendiabetes betrachtet Ungefähr 17 Millionen amerikanischer Erwachsener haben Diabetes Typ II.

Hinsichtlich der Kinder- und Jugendlichenbevölkerung hat die American Obesity Association darauf hingewiesen, dass dies ein ernsthaftes Problem ist, das nicht weniger als 20 - 22 % der Jugendlichen (Alter: 12 - 19 Jahre) und 19 % der Kinder (Alter: 6 bis 11 Jahre) betrifft. Dieser Trend steigt mit einer sehrbesorgniserregenden Geschwin-digkeit und gerät in manchen Ländern außer Kontrolle.

Es ist Ober eine Reihe von Todesfällen, die der Fettleibigkeit von Kindern zugeschrieben wurden, berichtet worden: Ein kürzlicher Fall in Großbritannien eines drei Jahre alten Kinds, das an Herzversagen starb, nachdem es grob übergewichtig worden war, und vier weitere Fälle von Kindern, die in der Nacht Hilfe beim Atmen brauchten, da sie so übergewichtig waren, dass ihnen das eigene Fett die Luft abschnürte.

Obwohl die bei Kindern und Jugendlichen verursachte Sterblichkeitsrate noch immer niedrig ist, hat die Fettleibigkeit von Kindern und Jugendlichen viele gesundheitliche und soziale Konsequenzen, die sich oft im Erwachsenenalter fortsetzen. Kinderarzte und Kinderfettleibigkeit erforschende Fachmänner melden häufigere Fälle von mit Fettleibigkeit zusammenhängenden Erkrankungen wie Typ-2-Diabetes, Asthma und Hypertonie, die einstmals als in der Vergangenheit sehr seltene Erwachsenenleiden betrachtet wurden.

Im menschlichen Gehirn gibt es drei Zentren, die die Nahrungsaufnahme regulieren: das sich im ventrolateralen Nukleus des Hypothalamus befindende Hungerzentrum, das sich im Hirmstamm befindende Appetitzentrum und das Sättigungszentrum im ventromedialen Hypothalamus. Das Hunger- und das Appetitzentrum stimulieren eine Person dazu zu essen, während das Sättigungszentrum das Bedürfnis nach Nahrung abschaltet.

Viele Einzelheiten dieses kompletten Regulationssystems werden bisher noch nicht begriffen. Es ist jedoch bekannt, dass die Nahrungsaufnahme sowohl von chemischen als auch sensorischen Signalen zwischen dem Gehlm und dem Rest des Körpers reguliert wird. Mund, Magen, Eingeweide und Nährstoffkonzentration sind Faktoren, die zum Appetit und zur Nahrungsaufnahme beitragen.

Die Hunger- und Sättigungsmechanismen werden von Sinnesempfindungen moduliert, die von Sinnesnervenden hervorgerufen werden, die sich in der Nähe des Lumens des Magen-Darm-Trakts befinden, und von neurologischen Mechanismen, die von schädlichen Stimuli ausgelöst werden.

Die Magen- und Darmnerven reagieren auf verschiedene Arten von Stimuli, einschließlich pH, chemische Zusammensetzung des Lumeninhalts oder Störung der Schleimhaul Während man isst und beim Verdauen von Nahrung erfassen die Sinnessysteme des Menschen forttaufend Veränderungen der chemischen und physischen Umgebung im Magen-Darm-Trakt und die Informationen dieser Veränderungen aktivieren das Hypothalamus- und das Himstammzentrum.

Die Pharmessen Scientific Inc. hat sich vor kurzem auf die Feineinstellung der Modulationsmechanismen konzentriert, die die Nahrungsaufnahme und Fettvertirennung steuern. Es wurden die Manipulation der viszeralen Empfindlichkeit und Wänneregulation erforscht, anstelle von hormonellen oder anderen, traditionelleren Ansätzen zum Gewichtsverlust, die den Hunger durch Fördern des ZNS oder Sympathikusstimulierung mildem.

Es wurden neuartige Behandlungen zur Gewichtskontroile identifiziert, die eine Reihe von natürlichen Quellen ätherischer Öle anwenden, die eine hohe Konzentration von zur Vanilloid-Klasse gehörenden Verbindungen enthalten. Diese chemischen Verbindungen haben eine einzigafige Spezifität, Sinnesfidsein des Magens zu binden, die normalerweise den verschiedenen Gehimzentren Informationen zum Magenzustand berichten.

Die Phannessen Scientific Inc. hat festgestellt, dass Vaniliolde zwei unerwartete und nicht nahe liegende Wirkungen haben, die zum Reduzieren des Gewichts dienen: Eine verschiebt das periphere Wärmeregulationsgleichgewicht hin zur verstärkten Thermogenese, was in einer Steigerung des Fettkatabolismus (Oxidation) im Außenbereich resultiert, gefolgt von Wärmeableitung, und eine zweite wirkt sich direkt auf Himzellen, die die Nahrungsaufnahme regulieren, Körpergewicht und Wärmeregulation aus, ohne Stimulierung des zentralen Nervensystems. Man ist bei den an diesen Ereignissen beteiligten Mechanismen noch immer dabei, diese zu begreifen, die Vorteile sind jedoch trotzdem therapeutisch relevant.

Die vorliegende Erfindung betrifft die Verwendung von mehreren natorlichen Quellen ätherischer Öle, einschließlich Zimtblattöl, Ingweröl und Kuricumaöl, die reich an Phenylalkenen, Gingerol bzw. Curcumin sind. Diese chemischen Verbindungen liegen in Pflanzen der Familien Lauraceae und Zingiberaceae vor. Am beachtenswertesten sind *Cinnamomum verum* J. Presl. (Zimt). *Zingiber officinalis* Roscoe (Ingwer) und *Curcuma domestica* Val (Kurkuma), die In der menschlichen Ernährung reichlich verzehrt werden, insbesondere in China, Indien und Pakistan, in denen die menschlichen Erwachsenenbevölkerungen traditionell schlank sind.

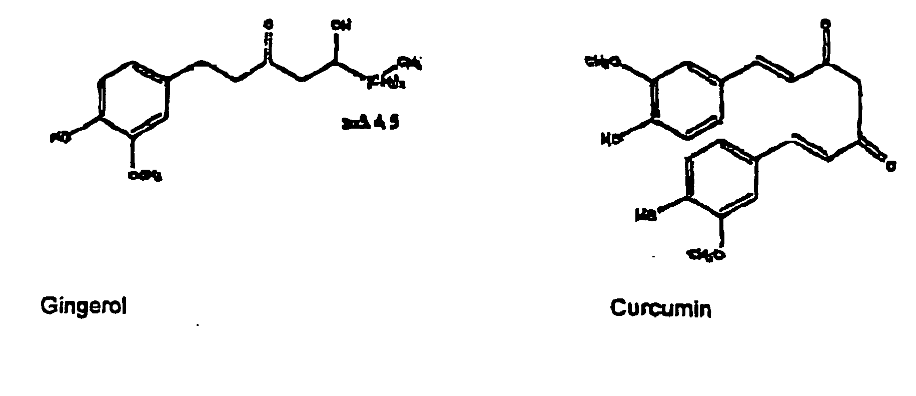

Der Zimtbaum ist ein kleiner Baum, der ursprünglich aus Indien stammt und auf den Inseln des Indischen Ozeans und in Südostasien eingeführt wurde und derzeit hauptsächlich in Sri Lanka angebaut wird. Er wird seit 4000 Jahren aufgrund seiner medizinischen und kulinarischen Eigenschaften verwendet. Von Zimt ist als ein Stomachikum und Karminativum bei leichten Magen-Darm-Krämpfen berichtet worden.

Ingwer ist seit mehr als 25 Jahrhunderten bei der Formulierung von unzähligen traditionellen chinesischen Heilmitteln verwendet worden, bei Magen-Darm-Beschwerden und als ein Mittel gegen Übelkeit. Mehrere klinische Prüfungen zeigen die Gefahrlosigkeit von Ingwer und Kurkuma in Menschen.

Kurkuma ist als eine gelbe Lebensmittelfarbe, Gewürz und Arzneimittel verwendet werden, was fast 4000 Jahre zur Vedic-Kultur in Indien zurückgeht, in der Kurkuma das Hauptgewürz war. Im Jahr 1280 beschrieb Marco Polo Kurkuma als "eine Pflanze mit den Eigenschaften von Safran, trotzdem ist es aber nicht wirklich Safran". Kurkuma ist in Asien innerlich als ein Arzneimittel für Zustände verwendet worden, darunter Kopfschmerzen, Magen- und Leberleiden. Kurkuma wird auch äußerlich angewendet, um Entzündungen zu heilen und als ein Kosmetikum.

Es ist eindeutig, dass keine der für Zimtblattöl, Ingwer- und Kurkumaöle berichteten medizinischen Wirkungen mit Gewichtskontrolle, Fettverbrennung oder Kontrolle von Fettleibigkeit in Verbindung gebracht worden ist. Die Pharmessen Scientific Inc. hat jedoch eine nicht nahe liegende Verbindung zwischen den scharfen Wirkungen dieser Öle auf die Magen- und Darmnervenfasern und ihrer Fähigkeit zur Kontrolle des Gewichts in Säugern gefunden.

Diese Öle wurden in einer Reihe von In-vitro-Assays bewertet und es wurde festgestellt, dass sie eine ausgeprägte Beziehung zwischen Struktur und Wirksamkeit auf die Hohlorgane von Säugern aufzeigen, insbesondere auf den Fundus der Ratte und den Vas deferens der Ratte.

Man war dazu in der Lage, IC₅₀-Werte für Eugenol, Gingerole und Curcuminoide und eine Beurteilung der Schärfe in einem Assay an menschlicher Zunge zu korrelieren. Außerdem hat man die Affinität dieser chemischen Verbindungen für Nervenfasern und ihre Fähigkeit zur Bewirkung von Hungerreduzierung, Thermogenese und Gewichtsreduktion in Versuchstieren korreliert.

Es wurden therapeutische Dosen der Öle an menschlichen Freiwilligen über einen Zeitraum von drei bis sechs Monaten geprüft und es wurde festgestellt, dass sie die Nahrungsaufnahme in Menschen reduzieren, was in einer allmählichen und anhaltenden Abnahme des Körpergewichts resultiert. Es wurden weitere Beobachtungen an Versuchsratten vorgenommen, insbesondere wenn diese chemischen Verbindungen mit Zitronensäure, Zitrusöl oder Zitrusextrakten kombiniert wurden.

Die Zusammensetzung war frei von Nebenwirkungen. Die Gewichtsreduktionsvorteile wurden verstärkt, wenn der Kakaoextrakt Proanthocyanidine (PAC) und Catechine enthielt. Es wurde eine Hypothese aufgestellt, dass PAC und Catechin die Fettaufnahme in Fettzellen unterdrückt. Kakao hat außerdem den Vorzug, eine vernachlässigbare Menge Koffein aufzuweisen, viel weniger als entkoffeinierter Kaffee, und bei seinem Hauptbestandteil Theobromin handelt es sich um ein Xanthin, das weniger ausgeprägte Auswirkungen auf das Herz-Kreislauf-System hervorruft.

Die Theobromin-Konzentration in Kakaoextrakt beträgt 6 - 8 %, was in einer Dosis von 50 - 100 mg 3 - 4 mg liefert, nicht genug, um ein wahrnehmbares Hirnstimulansereignis zu bewirken; es wurde jedoch festgestellt, dass diese Konzentration zum Fördern der Absorption anderer Bestandteile und zum Beschleunigen des Zellstoffwechsels ausreicht.

Mit dieser Formel behandelte Tiere zeigen normales Verhalten oder mehrdeutige Zeichen von ZNS-Stimulierung auf, wie bei Verwendung eines mehrdimensionalen Tier-Screenings demonstriert wurde. Es wurde theoretisiert, dass die Kombination von Theobromin, Catechinphenolen und Phenylethanolamin einen unerwarteten Stimmungshebungseffekt bewirkt, der in diesem Fall durch eine gute Tier- und Patienten-Compliance mit der Formel reflektiert werden kann.

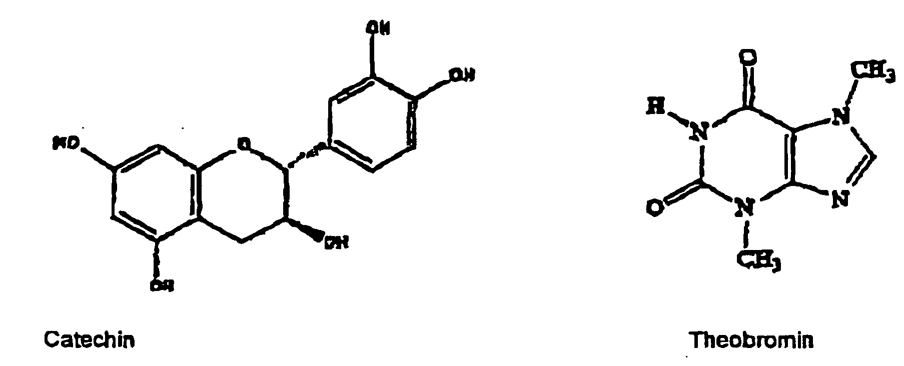

Eine wichtige und wertvolle Komponente der vorliegenden Erfindung ist das Formulierungsverfahren. Wenn man ätherische Öle kombiniert, reagieren die Vanilloid-Bestandteile miteinander und dies führt zu unerwünschten Veränderungen der chemischen Zusammensetzung, Wirksamkeit und Bioverfügbarkeit der Öle. Damit die Formel wirksam ist, ist es erforderlich, ein nicht nahe liegendes und unerwartetes Verfahren anzuwenden, bei dem Zimtöl, Ingweröl und Kurkumaöl, -extrakte und -oleoresine einzeln in Olivenöl eingekapselt und dann in pyrogene Kieselsäure eingearbeitet werden.

Der Einkapselungsvorgang bringt die individuelle Auflösung von mit Vanilloid angereicherten ätherischen Ölen in Olivenöl, Einarbeitung von Tröpfchen aus Öl/ätherischem Öl in unbehandelte pyrogene Kieselsäure mit sich, gefolgt von Pulvermischen unter Befolgung einer speziellen Mischungsmethodik zur sequenziellen Zugabe aller Inhaltsstoffe, um die endgültige pharmazeutische Dosisform herzustellen.

Die Tablettentechnologie ist aufgrund der folgenden unerwarteten und nicht nahe liegende Situation erforderlich, die zur Vermischung von Vanilloid-Mischungen führt.
1. Es wurde festgestellt, dass die ätherischen Öle oder in Zimtblattöl, Kurkuma und Ingwer vorliegenden reinen Vanilloide miteinander reagieren können, was in der Inaktivierung der separaten Bestandteile resultiert.
2. Die Reaktivität von Vanilloiden resultiert zum Teil aus der Gegenwart einer phenolischen (sauren) OH-Gruppe und Möglichkeit einer Moleküladdition, die zum Verlust eines Wassermoleküls führt.
3. Vanilloide sind in Wasser unlöslich und bedingen die Einarbeitung in eine Olivenölmatrix, um die Mizellenbildung zu erleichtern und die Abgabe und die Absorptionsraten zu steuern.

Mit Vanilloid angereicherte Öle und Extrakte werden einzeln in einem Olivenöltröpfchen eingefangen, der in einer Matrix aus hochgereinigter und unbehandelter pyrogener Kieselsäure eingebettet ist. Unbehandelte pyrogene Kieselsäure hat eine äußerst kleine Teilchengröße, geringe Schüttdichte und weist eine stark verzweigte kettenähnliche Struktur vor.

Wenn unbehandelte pyrogene Kieselsäure mit dem Olivenöl in Berührung kommt, ballen sie sich zusammen und bilden ein dreidimensionales Netzwerk aus Kieselsäure und Öltröpfchen. Zwischen den Kieselsäureaggregaten bilden sich große Hohlräume. Olivenöl füllt diese Hohlräume aus. Infolgedessen wird eine stark verzweigte Struktur gebildet, die eine effizientere Kontrolle der Verdickung und Rheologie bereitstellt. Die Mischung aus Olivenöl, ätherischem Öl und pyrogener Kieselsäure wird 3 min bei 3000 U/min gemischt, was die vollständige Integration der Vanilloide in der Formulierung ermöglicht.

Die Oberflächenchemie unbehandelter pyrogener Kieselsäure kann für diese Formulierung von Bedeutung sein. Die Oberfläche pyrogener Kieselsäure ist hydrophil (wasseranziehend) und ist zur Wasserstoffbrückenbindung mit den OH-Gruppen der Vanilloide in der Lage. Während der Formulierung dieses Produkts können sich Hydroxylgruppen der Vanilloide an einige der Siliziumatome auf der Teilchenoberfläche anheften. Das pyrogene Kieselsäuregel, das die Tröpfchen eines einzigen ätherischen Öls in seinem Inneren enthält, wird dann mit dem anderen pyrogenen Kieselsäuregel gemischt, das eine andere Art von ätherischen Ölen enthält. Das Gemisch aus Kieselsäure und Öl wird eines nach dem anderen zugegeben, so dass sie nicht miteinander reagieren, und dann mit Methylcellulose vermischt und zu Tabletten verpresst.

Die Tablettenformulierung von Zimtöl, Ingweröl und Kurkumaöl oder -oleoresinen kann durch die Verwendung von Zitronensäure verbessert werden, einem guten natürlichen Konservierungsstoff, der Lebensmitteln einen sauren Geschmack gibt und das Konservieren der Zusammensetzung unterstützt und eine Fettspeicherung verhindert.

Die Tabletten können auch durch eine Kombination von alpha-Amylase-Inhibitoren verbessert werden, die den Abbau von Stärke blockieren, wodurch die Aufnahme freien Zuckers verringert wird, was sich in einer reduzierten Kalorienaufnahme aus Kohlenhydraten und geringer Fettansammlung in Fettzellen ausdrückt. Alpha-Amylase-Inhibitoren beinhalten Proteinfraktionen aus Kidneybohne, Weizen und Kartoffel, um nur einige zu nennen. Die alpha-Amylase-Menge bietet die Möglichkeit, 500-mg- oder 1000-mg-Tabletten zu erzeugen, die für Erwachsene und Jugendliche verträglich sind.

Diese Tablette bleibt äußerst hygroskopisch, was die schnelle Auflösung der Tablette und Freisetzung des Inhalts im gastrischen Medium ermöglicht. Zusätzliche Trägerstoffe für diese Formel beinhalten Methylcellulose, kristalline Cellulose 101 und kristalline Cellulose 102 als auch Propylmethylcellulose.

Ein weiterer Formulierungsteil dieser Erfindung ist das Verfahren zur Herstellung eines Sirups, der insbesondere von Kindern verwendet werden soll. Die Sirupformulierung beinhaltet Curcumin, Zimtblattöl und Kakaoextrakt in einer Basis aus destilliertem Wasser mit Sorbit als Konservierungsstoff und natürlichen Farb- und Geschmackszusätzen.

Zusammenfassend ist die vorliegende Erfindung auf ein Verfahren zur Formulierung von ätherischen Ölen und pharmazeutischen Zusammensetzungen aus Zimtblattöl, Ingweröl und Kurkumaöl ausgerichtet. Die Zusammensetzungen können als schnell wirkende oder Retardtabletten und ein Sirup formuliert werden, wobei die Formulierungen zur Kontrolle des Gewichts und Förderung des Fettstoffwechsels in Erwachsenen, Kindern und Jugendlichen wirksam sind.

Die Zusammensetzungen rufen eine Verringerung der viszeralen Empfindlichkeit hervor, die sich in einer Portionskontrolle und einer Reduzierung der Kalorienaufnahme ausdrückt. Zusätzlicher Gewichtsverlust wird mittels Lipolyse und Glukoneogenese erzielt, eine Reduzierung der Fettaufnahme und -speicherung und eine Kohlenhydratblockierung durch die Kombination von Catechinen und Proanthocyanidinen in anderen Inhaltsstoffen wie Kakao, Zitronensäure und anderen Bestandteilen in der Formel.

Es ist eine primäre Aufgabe der vorliegenden Erfindung, ein Formulierungsverfahren bereitzustellen, in dem Zimtblattöl, Gingerol und Curcumin bei Kombination mit unbedenklichen Trägerstoffen mit Arzneimittelgüte die Wechselwirkung von Phenylgruppen verringern und in Tabletten- und Sirupform als ein vor Mahlzeiten einzunehmendes natürliches Nahrungsergänzungsmittel dargereicht werden. Diese Ergänzungsmittel rufen eine Wärmeerzeugung, verstärkten Fettstoffwechsel und Oxidation hervor, gefolgt von Wärmeableitung, was eine Kontrolle des Gewichts von Erwachsenen, Jugendlichen und Kindern ermöglicht.

### BEISPIELE

Eine pharmazeutische Zusammensetzung aus Zimtblattöl, Ingweröl mit einer hohen Konzentration an Gingerolen und Kurkumaextrakt mit einer hohen Curcuminkonzentration wird mit Olivenöl gemischt und die inhaltsstoffe werden in einer Tablette zubereitet, kombiniert mit einem oder allen der folgenden Stoffe: Zitronensäure, Zitronenöl, Kakaoextrakte und einer Proteinfraktion aus Weizen oder Kidneybohne mit alpha-Amylase-Inhibitoren, wie im Folgenden beschrieben. Die folgenden Beispiele sind Beispiele von Tablettenformulierungen des Produkts.

### TABLETTEN

| Langsam wirkende Formulierung mit hoher Konzentration an Zimtblattöl | |
|---|---|
| Inhaltsstoffe | Menge pro Tablette |
| | (1 - 15 g) |
| Zimtblattöl (90 % Eugenol) | 10 - 20 mg |
| Ingweröl (20 % Gingerale) | 1 - 7 mg |
| Kurkumaöl (95 % Curcumin) | 0.5 - 3 mg |
| Olivenöl | 3 -10 mg |
| Kakaoextrakt (6 % Theobromin) | 50 - 100 mg |
| Zitronensäure oder Zitrusöl | 1 - 5 mg |
| Proteinfraktion aus Kidneybohne oder Weizen | 500 - 1000 mg |
| Pyrogene Kieselsäure | 25 - 50 mg |
| Avicel PH 102 | 100 - 200 mg |
| Avicel PH 101 | 150 - 350 mg |
| Methocel | 100 - 200 mg |

| Schnell wirkende Formulierung mit hoher Konzentration an Zimtblattöl | |
|---|---|
| Inhaltsstoff | Menge pro Tablette (1 - 15 g) |
| Zimtblattöl (90 % Eugenol) | 10 - 20 mg |
| Ingweröl (30 % Gingerole) | 1 - 7 mg |
| Kurkumaöl (95 % Curcumin) | 0,5 - 3 mg |
| Olivenöl | 3 - 10 mg |
| Kakaoextrakt (6 % Theobromin) | 50 - 100 mg |
| Zitronensäure oder Zitrusöl | 1 - 5 mg |
| Proteinfraktion aus Kidneybohne oder Weizen | 500 - 1000 mg |
| Avicel PH 102 | 150 - 250 mg |
| Avicel PH 101 | 400 - 600 mg |
| Pyrogene Kieselsäure | 25 - 50 mg |

| Langsam wirkende Formulierung mit geringer Konzentration an Zimtblattöl | |
|---|---|
| Inhaltsstoff | Menge pro Tablette (1 - 15 g) |
| Ingweröl (30 % Gingerole) | 10 - 20 mg |
| Kurkumaöl (95 % Curcumin) | 1 - 7 mg |
| Zimtblattöl (90 % Eugenol) | 5 - 10 mg |
| Olivenöl | 3 - 10 mg |
| Kakaoextrakt (6 % Theobromin) | 50 - 100 mg |
| Zitronensäure oder Zitrusöl | 1 - 5 mg |
| Proteinfraktion aus Kidneybohne oder Weizen | 500 - 1000 mg |
| Pyrogene Kieselsäure | 25 - 50 mg |
| Avicel PH 102 | 100 - 200 mg |
| Avicel PH 101 | 150 - 350 mg |
| Pyrogene Kieselsäure | 100 - 200 mg |

| Schnell wirkende Formulierung mit geringer Konzentration an Zimtblattöl | |
|---|---|
| Inhaltsstoff | Menge pro Tablette (1 - 20 g) |
| Ingweröl (30 % Gingerole) | 10 - 20 mg |
| Kurkumaöl (95 % Curcumin) | 1 - 7 mg |
| Zimtblattöl (90 % Eugenol) | 5 - 10 mg |
| Olivenöl | 3 - 10 mg |
| Kakaoextrakt (6 % Theobromin) | 50 - 100 mg |
| Zitronensäure oder Zitrusöl | 1 - 5 mg |
| Alpha-Amylase aus Kidneybohne | 500 - 1000 mg |
| Avicel PH 102 | 150 - 250 mg |
| Avicel PH 101 | 400 - 600 mg |
| Pyrogene Kieselsäure | 25 - 50 mg |

| Schnell wirkende Formulierung mit geringer Konzentration an Zimtblattöl für Jugendliche Kleinere Tabletten | |
|---|---|
| Inhaltsstoff | Menge pro Tablette (1 - 2,0 g) |
| Ingweröl (30 % Gingerole) | 5 - 17 mg |
| Kurkumaöl (95 % Curcumin) | 1 - 7 mg |
| Zimtblattöl (90 % Eugenol) | 2 - 5 mg |
| Olivenöl | 3 - 10 mg |
| Kakaopulver | 50 - 100 mg |
| Alpha-Amylase aus Kidneybohne | 300 - 800 mg |
| Avicel PH 102 | 100 - 250 mg |
| Avicel PH 101 | 350 - 600 mg |
| Pyrogene Kieselsäure | 20 - 50 mg |

Eine pharmazeutische Zusammensetzung in Sirupform für Kinder umfässt therapeutisch wirksame Mengen Zimtblattöl, Kurkumaextraktöl oder -oleoresin und Kombinationen dieser Öle mit Kakaopulver, Zitronensäure, ätherischen Zitrusölen und einer Proteinfraktion aus weißer Kidneybohne mit alpha-Amylase-Inhibifionswirkung.

### SIRUP

Sirupformutierung für Kinder mit geringer Konzentration an Zimtblattöl und ohne Ingwer, um eine chemische Reaktion zu vermeiden

| Inhaltsstoff | Prozentanteil im Sirup |
|---|---|
| Kurkumapulver (95 % Curcumin) | 2,0 - 7,0 |
| Zimtblattöl (90 % Eugenol) | 2,0 - 5,0 |
| Olivenöl | 0,2 - 1,0 |
| Kakaoputver | 1,0 - 10,0 |
| Alpha-Amylase aus Kidneybohne | 5,0 - 25,0 |
| Kirsch- oder Traubengeschmackstoff | 0,5 - 1,0 |
| Natürlicher Farbstoff | 0,2 - 0,5 |
| Sorbit | 6,0 - 10,0 |
| Destilliertes Wasser | 40,0 - 70,0 |

## Patentansprüche

1. Pharmazeutische Zusammensetzung zur oralen Verabreichung und zur Verwendung bei der Behandlung von Übergewicht beim Menschen oder Säugetier, wobei die pharmazeutische Zusammensetzung ein oder mehrere Vanilloide, Carvacrol und/oder pharmazeutisch verträgliche Salze davon als Komponente a) umfasst, wobei Komponente a) aus der Gruppe ausgewählt wird, die aus Eugenol, Gingerol, Curcumin und Carvacrol besteht, **dadurch gekennzeichnet, dass** eine Dosierungseinheit in Tablettenform
| | |
|---|---|
| 2 - 20 mg | Zimtblattöl (90 % Eugenol) |
| 1 - 20 mg | Ingweröl (20 % - 30 % Gingerole) |
| 0,5 - 7 mg | Kurkumaöl (95 % Curcumin) |
| 3 - 10 mg | Olivenöl |
| 20 - 200 mg | Pyrogene Kieselsäure, |
umfasst, wobei sich alle Gewichtsangaben auf 1000 - 500 mg der Tablette beziehen.

2. Pharmazeutische Zusammensetzung zur oralen Verabreichung und zur Verwendung bei der Behandlung von Übergewicht beim Menschen oder Säugetier, wobei die pharmazeutische Zusammensetzung ein oder mehrere ätherische Öle, die ein oder mehrere Vanilloide, Carvacrol und/oder pharmazeutisch verträgliche Salze davon enthalten, als Komponente a) umfasst, wobei Komponente a) aus der Gruppe ausgewählt wird, die aus Zimtblattöl, ingwerextrakt, -oleoresin oder -öl,
Kurkumaextraktöl oder-oleoresin sowie Thymianöl besteht, **dadurch gekennzeichnet, dass** eine Dosierungseinheit in Tablettenform
| | |
|---|---|
| 2 - 20 mg | Zimtblattöl (90 % Eugenol) |
| 1 - 20 mg | Ingweröl (20 % - 30 % Gingerole) |
| 0,5 - 7 mg | Kurkumaöl (95 % Curcumin) |
| 3 - 10 mg | Olivenöl |
| 20 - 200 mg | Pyrogene Kieselsäure, |
umfasst, wobei sich alle Gewichtsangaben auf 1000 - 1500 mg der Tablette beziehen.

3. Pharmazeutische Zusammensetzung zur oralen Verabreichung und zur Verwendung bei der Behandlung von Übergewicht beim Menschen oder Säugetier, wobei die pharmazeutische Zusammensetzung ein oder mehrere Vanilloide, pharmazeutisch verträgliche Salze davon als Komponente a) und ein pharmazeutisch verträgliches Vehikel als Komponente b) umfasst, wobei Komponente a) aus der Gruppe ausgewählt wird, die aus Eugenol, Gingerol, Curcumin und Carvacrol besteht, **dadurch gekennzeichnet, dass** Komponente b) pyrogene kieselsäure ist.

4. Pharmazeutische Zusammensetzung nach Anspruch 3, **dadurch gekennzeichnet, dass** eine Dosierungseinheit in Tablettenform
| | |
|---|---|
| 2 - 20 mg | Zimtblattöl (90 % Eugenol) |
| 1 - 20 mg | Ingweröl (20 % - 30 % Gingerole) |
| 0,5 - 7 mg | Kurkumaöl (95 % Curcumin) |
| 3 - 10 mg | Olivenöl |
| 20 - 200 mg | Pyrogene Kieselsäure, |
umfasst, wobei sich alle Gewichtsangaben auf 1000 - 1500 mg der Tablette beziehen.

5. Pharmazeutische Zusammensetzung zur oralen Verabreichung und zur Verwendung bei der Behandlung von Übergewicht beim Menschen oder Säugetier, wobei die pharmazeutische Zusammensetzung ein oder mehrere ätherische Öle, die ein oder mehrere Vanilloide, Carvacrol und/oder pharmazeutisch verträgliche Salze davon enthalten, als Komponente a) und ein pharmazeutisch verträgliches Vehikel als Komponente b) umfasst, wobei Komponente a) aus der Gruppe ausgewählt wird, die aus Zimtblattöl, Ingwerextrakt, -oleoresin oder -öl, Kurkumaextraktöl oder -oleoresin sowie Thymianöl besteht, **dadurch gekennzeichnet, dass** Komponente b) pyrogene Kieselsäure umfasst.

6. Pharmazeutischen Zusammensetzung nach Anspruch 5, **dadurch gekennzeichnet, dass** eine Dosierungseinheit in Tablettenform
| | |
|---|---|
| 2 - 20 mg | Zimtblattöl (90 % Eugenol) |
| 1 - 20 mg | Ingweröl (20 % - 30 % Gingerole) |
| 0,5 - 7 mg | Kurkumaöl (95 % Curcumin) |
| 3 - 10 mg | Olivenöl |
| 20 - 200 mg | Pyrogene Kieselsäure, |
umfasst, wobei sich alle Gewichtsangaben auf 1000 - 1500 mg der Tablette beziehen.

7. Pharmazeutische Zusammensetzung nach einem der Oberbegriffe der Ansprüche 1 bis 3 oder 5, **dadurch gekennzeichnet, dass** eine Dosierungseinheit in Sirupform umfasst:
| | |
|---|---|
| 2,0 - 7,0 % | Kurkumaöl (95 % Curcumin) |
| 2,0 - 5,0 % | Zimtblattöl (90 % Eugenol) |
wobei sich alle Prozentangaben auf Anteile im Sirup beziehen.

8. Pharmazeutische Zusammensetzung nach einem der Ansprüche 2, 5 oder 6 oder nach Anspruch 7, soweit dieser auf die Ansprüche 2 oder 5 rückbezogen ist, **dadurch gekennzeichnet, dass** das ätherische Öl eine hohe Konzentration, vorzugsweise >30 Gew.%, der zur Klasse der Vanilloide gehörenden Verbindungen aufweist.

9. Pharmazeutische Zusammensetzung nach einem der Ansprüche 3 bis 6 oder nach Anspruch 7, soweit dieser auf die Ansprüche 3 oder 5 rückbezogen ist, **dadurch gekennzeichnet, dass** das Vehikel wenigstens ein Pflanzenöl, nämlich Olivenöl, umfasst.

10. Pharmazeutische Zusammensetzung nach einem der Ansprüche 1 bis 9 oder nach einem der Oberbegriffe der Ansprüche 1 bis 3 oder 5, **dadurch gekennzeichnet, dass** die pharmazeutische Zusammensetzung als weitere Komponente Kakaoextrakt, vorzugsweise mit einem Theobromingehalt von 6 - 8 %, umfasst.

11. Pharmazeutische Zusammensetzung nach einem der Ansprüche 1 bis 10 oder nach einem der Oberbegriffe der Ansprüche 1 bis 3 oder 5, **dadurch gekennzeichnet, dass** die pharmazeutische Zusammensetzung als weitere Komponente ein oder mehrere Proanthocyanidine umfasst.

12. Pharmazeutische Zusammensetzung nach einem der Ansprüche 1 bis 11 oder nach einem der Oberbegriffe der Ansprüche 1 bis 3 oder 5, **dadurch gekennzeichnet, dass** die pharmazeutische Zusammensetzung als weitere Komponente ein oder mehrere Catechine umfasst.

13. Pharmazeutische Zusammensetzung nach einem der Ansprüche 1 bis 12 oder nach einem der Oberbegriffe der Ansprüche 1 bis 3 oder 5, **dadurch gekennzeichnet, dass** die pharmazeutische Zusammensetzung als weitere Komponente Phenylethanolamin umfasst.

14. Pharmazeutische Zusammensetzung nach einem der Ansprüche 1 bis 13 oder nach einem der Oberbegriffe der Ansprüche 1 bis 3 oder 5, **dadurch gekennzeichnet, dass** die chemische Zusammensetzung als weitere Komponente Zitronensäure, wenigstens ein ätherisches Zitrusöl und/oder wenigstens ein Zitrusextrakt umfasst.

15. Pharmazeutische Zusammensetzung nach einem der Ansprüche 1 bis 14 oder nach einem der Oberbegriffe der Ansprüche 1 bis 3 oder 5, **dadurch gekennzeichnet, dass** die chemische Zusammensetzung als weitere Komponente einen Stoff oder ein Stoffgemisch mit alpha-Amylase-Inhibitionswirkung, nämlich eine Proteinfraktion aus der weißen Kidneybohne, aus Weizen oder aus der Kartoffel, umfasst.

16. Pharmazeutische Zusammensetzung nach Anspruch 1, 2, 4 oder 6, **dadurch gekennzeichnet, dass** die Dosierungseinheit in Tablettenform als weitere Komponente umfasst:
| | |
|---|---|
| 50 - 100 mg | Kakaoextrakt (6 % Theobromin) |
| und/oder | |
| 1 - 5 mg | Zitronensäure oder Zitrusöl |
| und/oder | |
| 300 - 1000 mg | Proteinfraktion aus Kidneybohne oder Weizen, |
wobei sich alle Gewichtsangaben auf 1000 - 2000 mg der Tablette beziehen.

17. Pharmazeutische Zusammensetzung nach Anspruch 7, **dadurch gekennzeichnet, dass** die Dosierungseinheit in Sirupform als weitere Komponente destilliertes Wasser umfasst.

18. Pharmazeutische Zusammensetzung nach Anspruch 7 oder 17, **dadurch gekennzeichnet, dass** die Dosierungseinheit in Sirupform als weitere Komponente umfasst:
| | |
|---|---|
| 0,2 - 1,0 % | Olivenöl |
| und/oder | |
| 1,0 - 10,0 % | Kakaoextrakt (6 % Theobromin) |
| und/oder | |
| 5,0 - 25,0 % | Proteinfraktion aus Kidneybohne |
| und/oder | |
| 40,0 - 70,0 % | destilliertes Wasser, |
wobei sich alle Prozentangaben auf Anteile im Sirup beziehen.

19. Verwendung einer pharmazeutischen Zusammensetzung nach einem der Ansprüche 1 bis 18 zur Herstellung eines Medikaments und/oder eines Nahrungsergänzungsmittels zur Behandlung von Übergewicht beim Menschen oder Säugetier.

20. Verfahren zur Herstellung einer pharmazeutischen Zusammensetzung in Tablettenform nach einem der Ansprüche 1 bis 6 oder 8 bis 16 mit folgenden Verfahrensschritten:
i) Vermischen eines oder mehrerer Vanilloide, Carvacrols und/oder pharmazeutisch verträglicher Salze davon, mit einem Pflanzenöl,
ii) Einarbeiten von Tröpfchen des im Verfahrensschritt i) hergestellten Gemisches In pyrogene Kieselsäure.

21. Verfahren zur Herstellung einer pharmazeutischen Zusammensetzung in Tablettenform nach einem der Ansprüche 1 bis 6 oder 8 bis 16, mit folgenden Verfahrensschritten:
i) Vermischen eines oder mehrerer ätherischer Öle, die ein oder mehrere Vanilloide, Carvacrol und/oder pharmazeutisch verträgliche Salze davon enthalten, mit einem Pflanzenöl,
ii) Einarbeiten von Tröpfchen des im Verfahrensschritt i) hergestellten Gemisches in pyrogene Kieselsäure.

22. Verfahren nach Anspruch 20 oder 21, **dadurch gekennzeichnet, dass** im Verfahrensschritt ii) hochgereinigte unbehandelte pyrogene Kieselsäure verwendet wird.

23. Verfahren nach einem der Ansprüche 20 bis 22, **gekennzeichnet durch** Verfahrensschritt
iii) Vermischen des im Verfahrensschritt ii) erhaltenen Produkts eines Vanilloids, Carvacrols und/oder pharmazeutisch verträglichen Salzes davon bzw. eines ätherischen Öls, das ein, oder mehrere Vanilloide, Carvacrol und/oder pharmazeutisch verträgliche Salze davon enthält, mit wenigstens einem weiteren im Verfahrensschritt ii) erhaltenen Produkts eines anderen Vanilloids, Carvacrols und/oder pharmazeutisch verträglichen Salzes davon bzw. eines anderen ätherischen Öls, das ein oder mehrere Vaniliolde, Carvacrol und/oder pharmazeutisch verträgliche Salze davon enthält.

24. Verfahren nach einem der Ansprüche 20 bis 23, **gekennzeichnet durch** einen weiteren Verfahrensschritt, bei welchem dem im Verfahrensschritt ii) oder iii) erhaltenen Produkt wenigstens ein zusätzlicher Trägerstoff zugemischt wird, der aus der Gruppe ausgewählt wird, die Methylcellulose, kristalline Cellulose 101, kristalline Cellulose 102 und Propylmethylcellulose umfasst.

25. Verfahren zur Herstellung einer pharmazeutischen Zusammensetzung in Sirupform nach einem der Ansprüche 7, 17 oder 18, mit folgendem Verfahrensschritt: j) Vermischen eines oder mehrerer Vanilloide, Carvacrol und/oder pharmazeutisch verträglicher Salze davon, mit einem Pflanzenöl.

26. Verfahren zur Herstellung einer pharmazeutischen Zusammensetzung in Sirupform nach einem der Ansprüche 7, 17 oder 18, mit folgendem Verfahrensschritt:
j) Vermischen eines oder mehrerer ätherischer Öle, die ein oder mehrere Vanilloide, Carvacrol und/oder pharmazeutisch verträgliche Salze davon enthalten, mit einem Pflanzenöl.

27. Verfahren nach Anspruch 25 oder 26, **gekennzeichnet durch** Verfahrensschritt k) Vermischen des im Verfahrensschritt j) erhaltenen Produkts eines Vanilloids, Carvacrols und/oder pharmazeutisch verträglichen Salzes davon bzw. eines ätherischen Öls, das ein oder mehrere Vanilloide, Carvacrol und/oder pharmazeutisch verträgliche Salze davon enthält, mit wenigstens einem weiteren im Verfahrensschritt j) erhaltenen Produkts eines anderen Vanilloids, Carvacrols und/oder pharmazeutisch verträglichen Salzes davon bzw. eines anderen ätherischen Öls, das ein oder mehrere Vanilloide, Carvacrol und/oder pharmazeutisch verträgliche Salze davon enthält.

28. Verfahren nach einem der Ansprüche 20 bis 27, **gekennzeichnet durch** einen weiteren Verfahrensschritt, bei welchem dem im Verfahrensschritt ii), iii), j) oder k) erhaltenen Produkt zusätzlich Zitronensäure, wenigstens ein ätherisches Zitrusöl und/oder wenigstens ein Zitrusextrakt zugemischt wird.

29. Verfahren nach einem der Ansprüche 20 bis 28, **gekennzeichnet durch** einen weiteren Verfahrensschritt, bei welchem dem im Verfahrensschritt ii), iii). j) oder k) erhaltenen Produkt zusätzlich Kakaoextrakt, vorzugsweise mit einem Theobromingehalt von 6 - 8 %, zugemischt wird.

30. Verfahren nach einem der Ansprüche 20 bis 29, **gekennzeichnet durch** einen weiteren Verfahrensschritt, bei welchem dem im Verfahrensschritt ii), iii), j) oder k) erhaltenen Produkt zusätzlich ein oder mehrere Catechine zugemischt werden.

31. Verfahren nach einem der Ansprüche 20 bis 30, **gekennzeichnet durch** einen weiteren Verfahrensschritt, bei welchem dem im Verfahrensschritt ii), iii), j) oder k) erhaltenen Produkt zusätzlich ein oder mehrere Proanthocyanidine zugemischt werden.

32. Verfahren nach einem der Ansprüche 20 bis 30, **gekennzeichnet durch** einen weiteren Verfahrensschritt, bei welchem dem im Verfahrensschritt ii), iii), j) oder k) erhaltenen Produkt zusätzlich Phenylethenoiamin zugemischt wird.

33. Verfahren nach einem der Ansprüche 20 bis 32, **gekennzeichnet durch** einen weiteren Verfahrensschritt, bei welchem dem im Verfahrensschritt ii), iii), j) oder k) erhaltenen Produkt zusätzlich ein Stoff oder Stoffgemisch mit alpha-Amylase-Inhibitionswirkung, nämlich eine Proteinfraktion aus der weißen Kidneybohne, aus Weizen oder aus der Kartoffel, zugemischt wird

## Claims

1. A pharmaceutical composition for oral administration and for use in the treatment of overweight in humans or mammals, wherein the pharmaceutical composition includes one or more vanilloids, carvacrol and/or pharmaceutically compatible salts thereof as component a), wherein component a) is selected from the group consisting of eugenol, gingerol, curcumin and carvacrol, **characterised in that** a unit dose in tablet form includes
| | |
|---|---|
| 2 - 20mg | cinnamon leaf oil (90% eugenol) |
| 1 - 20mg | ginger oil (20% - 30% gingerols) |
| 0.5 - 7mg | curcuma oil (95% curcumin) |
| 3 - 10mg | olive oil |
| 20 - 200mg | pyrogenic silicic acid, |
wherein all weight information relates to 1000 - 1500mg of the tablet.

2. A pharmaceutical composition for oral administration and for use in the treatment of overweight in humans or mammals, wherein the pharmaceutical composition includes one or more essential oils, which contain one or more vanilloids, carvacrol and/or pharmaceutically compatible salts thereof, as component a), wherein component a) is selected from the
group consisting of cinnamon leaf oil, ginger extract, oleoresin or oil, curcuma extract oil or oleoresin and thyme oil, **characterised in that** a unit dose in tablet form includes
| | |
|---|---|
| 2 - 20mg | cinnamon leaf oil (90% eugenol) |
| 1 - 20mg | ginger oil (20% - 30% gingerols) |
| 0.5 - 7mg | curcuma oil (95% curcumin) |
| 3 - 10mg | olive oil |
| 20 - 200 mg | pyrogenic silicic acid, |
wherein all weight information relates to 1000 - 1500mg of the tablet.

3. A pharmaceutical composition for oral administration and for use in the treatment of overweight in humans or mammals, wherein the pharmaceutical composition includes one or more vanilloids, pharmaceutically compatible salts thereof as component a) and a pharmaceutically compatible vehicle as component b), wherein component a) is selected from the group consisting of eugenol, gingerol, curcumin and carvacrol, **characterised in that** component b) is pyrogenic silicic acid.

4. The pharmaceutical composition according to Claim 3, **characterised in that** a unit dose in tablet form includes
| | |
|---|---|
| 2 - 20mg | cinnamon leaf oil (90% eugenol) |
| 1 - 20mg | ginger oil (20% - 30% gingerols) |
| 0.5 - 7mg | curcuma oil (95% curcumin) |
| 3 -10mg | olive oil |
| 20 - 200mg | pyrogenic silicic acid, |
wherein all weight information relates to 1000 - 1500mg of the tablet.

5. A pharmaceutical composition for oral administration and for use in the treatment of overweight in humans or mammals, wherein the pharmaceutical composition includes one or more essential oils, which contain one or more vanilloids, carvacrol and/or pharmaceutically compatible salts thereof, as component a) and a pharmaceutically compatible vehicle as component b), wherein component a) is selected from the group consisting of cinnamon leaf oil, ginger extract, oleoresin or oil, curcuma extract oil or oleoresin and thyme oil, **characterised in that** component b) includes pyrogenic silicic acid.

6. The pharmaceutical composition according to Claim 5, **characterised in that** a unit dose in tablet form includes
| | |
|---|---|
| 2 - 20mg | cinnamon leaf oil (90% eugenol) |
| 1 - 20mg | ginger oil (20% - 30% gingerols) |
| 0.5 - 7mg | curcuma oil (95% curcumin) |
| 3 - 10mg | olive oil |
| 20 - 200mg | pyrogenic silicic acid, |
wherein all weight information relates to 1000 - 1500mg of the tablet.

7. The pharmaceutical composition according to one of the preambles of the claims 1 to 3 or 5, **characterised in that** a unit dose in syrup form includes:
| | |
|---|---|
| 2.0 - 7.0% | curcuma oil (95% curcumin) |
| 2.0 - 5.0% | cinnamon leaf oil (90% eugenol) |
wherein all percentage information relates to proportions in the syrup.

8. The pharmaceutical composition according to one of claims 2, 5 or 6, or according to Claim 7, insofar as it relates to Claim 2 or 5, **characterised in that** the essential oil has a high concentration, preferably >30 wt.%, of the compounds belonging to the vanilloids class.

9. The pharmaceutical composition according to one of claims 3 to 6 or according to Claim 7, insofar as it relates to Claim 3 or 5, **characterised in that** the vehicle includes at least one vegetable oil, namely olive oil.

10. The pharmaceutical composition according to one of claims 1 to 9 or according to one of the preambles of the claims 1 to 3 or 5, **characterised in that** the pharmaceutical composition includes as a further component cocoa extract, preferably having a theobromine content of 6 - 8%.

11. The pharmaceutical composition according to one of claims 1 to 10 or according to one of preambles of the claims 1 to 3 or 5, **characterised in that** the pharmaceutical composition includes one or more proanthocyanidins as a further component.

12. The pharmaceutical composition according to one of claims 1 to 11 or according to one of the preambles of the claims 1 to 3 or 5, **characterised in that** the pharmaceutical composition includes one or more catechins as a further component.

13. The pharmaceutical composition according to one of claims 1 to 12 or according to one of the preambles of the claims 1 to 3 or 5, **characterised in that** the pharmaceutical composition includes phenylethanolamine as a further component.

14. The pharmaceutical composition according to one of claims 1 to 13 or according to one of the preambles of the claims 1 to 3 or 5, **characterised in that** the chemical composition includes citric acid, at least one essential citrus oil and/or at least one citrus extract as a further component.

15. The pharmaceutical composition according to one of claims 1 to 14 or according to one of the preambles of the claims 1 to 3 or 5, **characterised in that** the chemical composition includes a substance or a substance mixture with alpha-amylase inhibition effect, namely a protein fraction from white kidney bean, from wheat or from potato, as a further component.

16. The pharmaceutical composition according to Claim 1, 2, 4 or 6, **characterised in that** the unit dose in tablet form includes as a further component:
| | |
|---|---|
| 50 - 100mg | cocoa extract (6% theobromine) |
| and/or | |
| 1 - 5mg | citric acid or citrus oil |
| and/or | |
| 300 - 1000mg | protein fraction from kidney bean or wheat, |
wherein all weight information relates to 1000 - 2000mg of the tablet.

17. The pharmaceutical composition according to claim 7, **characterised in that** the unit dose in syrup form includes distilled water as a further component.

18. The pharmaceutical composition according to Claim 7 or 17, **characterised in that** the unit dose in syrup form includes as a further component:
0.2 - 1.0% olive oil
and/or
1.0 - 10.0% cocoa extract (6% theobromine)
and/or
5.0 - 25.0% protein fraction from kidney bean
and/or
40.0 - 70.0% distilled water,
wherein all percentage information relates to proportions in the syrup.

19. A use of a pharmaceutical composition according to one of claims 1 to 18 for the manufacture of a medication and/or a dietary supplement for treating overweight in humans or mammals.

20. A method for manufacturing a pharmaceutical composition in tablet form according to one of claims 1 to 6 or 8 to 16, with the following method steps:
i) mixing one or more vanilloids, carvacrols and/or pharmaceutically compatible salts thereof with a vegetable oil,
ii) introducing droplets of the mixture made in method step i) into pyrogenic silicic acid.

21. The method for manufacturing a pharmaceutical composition in tablet form according to one of claims 1 to 6 or 8 to 16, with the following method steps:
i) mixing one or more essential oils, which contain one or more vanilloids, carvacrol and/or pharmaceutically compatible salts thereof, with a vegetable oil,
ii) introducing droplets of the mixture made in method step i) into pyrogenic silicic acid.

22. The method according to Claim 20 or 21, **characterised in that** highly purified untreated pyrogenic silicic acid is used in the method step ii).

23. The method according to one of claims 20 to 22, **characterised by** method step
iii) mixing the product, obtained in method step ii), of a vanilloid, carvacrol and/or pharmaceutically compatible salt thereof or of an essential oil containing one or more vanilloids, carvacrol and/or pharmaceutically compatible salts thereof, with at least one further product, obtained in method step ii), of another vanilloid, carvacrol and/or pharmaceutically compatible salt thereof or of another essential oil containing one or more vanilloids, carvacrol and/or pharmaceutically compatible salts thereof.

24. The method according to one of claims 20 to 23, **characterised by** a further method step in which at least one additional carrier selected from the group including methyl cellulose, crystalline cellulose 101, crystalline cellulose 102 and propyl methyl cellulose is admixed with the product obtained in method step ii) or iii).

25. A method for manufacturing a pharmaceutical composition in syrup form according to one of claims 7, 17 or 18, with the following method step:
j) mixing one or more vanilloids, carvacrol and/or pharmaceutically compatible salts thereof with a vegetable oil.

26. A method for manufacturing a pharmaceutical composition in syrup form according to one of claims 7, 17 or 18, with the following method step:
j) mixing one or more essential oils, containing one or more vanilloids, carvacrol and/or pharmaceutically compatible salts thereof, with a vegetable oil.

27. The method according to Claim 25 or 26, **characterised by** method step
k) mixing the product, obtained in method step j), of a vanilloid, carvacrol and/or pharmaceutically compatible salt thereof or of an essential oil containing one or more vanilloids, carvacrol and/or pharmaceutically compatible salts thereof, with at least one further product, obtained in method step j), of another vanilloid, carvacrol and/or pharmaceutically compatible salt thereof, or of another essential oil containing one or more vanilloids, carvacrol and/or pharmaceutically compatible salts thereof.

28. The method according to one of claims 20 to 27, **characterised by** a further method step, in which citric acid, at least one essential citrus oil and/or at least one citrus extract is additionally admixed with the product obtained in method step ii), iii), j) or k).

29. The method according to one of claims 20 to 28, **characterised by** a further method step, in which cocoa extract, preferably with a theobromine content of 6 - 8%, is additionally admixed with the product obtained in method step ii), iii), j) or k).

30. The method according to one of claims 20 to 29, **characterised by** a further method step, in which one or more catechins are additionally admixed with the product obtained in method step ii), iii), j) or k).

31. The method according to one of claims 20 to 30, **characterised by** a further method step, in which one or more proanthocyanidins are additionally admixed with the product obtained in method step ii), iii), j) or k).

32. The method according to one of claims 20 to 30, **characterised by** a further method step, in which phenylethanolamine is additionally admixed with the product obtained in method step ii), iii), j) or k).

33. The method according to one of claims 20 to 32, **characterised by** a further method step, in which a substance or substance mixture with alpha-amylase inhibition effect, namely a protein fraction from white kidney bean, from wheat or from potato, is admixed with the product obtained in method step ii), iii), j) or k).

## Revendications

1. Composition pharmaceutique destinée à l'administration orale et à l'utilisation dans le cadre du traitement de l'excès pondéral chez l'homme ou chez les mammifères, la composition pharmaceutique comprenant un ou plusieurs vanilloïdes, du carvacrol et/ou des sels pharmaceutiquement compatibles de ceux-ci en tant que composant a), le composant a) étant choisi parmi le groupe constitué d'eugénol, de gingérol, de curcumine et de carvacrol, **caractérisée en ce qu'**une unité de dosage sous forme de comprimés comprend
| | |
|---|---|
| 2 à 10 mg | d'essence de feuilles du cannelier (90% d'eugénol) |
| 1 à 20 mg | d'essence de gingembre (20% à 30% de gingérols) |
| 0,5 à 7 mg | d'essence de curcuma (95% de curcumine) |
| 3 à 10 mg | d'huile d'olive |
| 20 à 200 mg | d'acide silique pyrogéné |
toutes les indications pondérales se rapportant à un comprimé de 1000 à 1500 mg.

2. Composition pharmaceutique destinée à l'administration orale et à l'utilisation dans le cadre du traitement de l'excès pondéral chez l'homme ou chez les mammifères, la composition pharmaceutique comprenant une ou plusieurs huiles essentielles, qui contiennent un ou plusieurs vanilloïdes, du carvacrol et/ou des sels pharmaceutiquement compatibles de ceux-ci en tant que composant a), le composant a) étant choisi parmi un groupe constitué d'essence de feuilles du cannellier, d'extrait, d'oléorésine ou d'essence de gingembre, d'huile d'extrait ou d'oléorésine de curcuma, ainsi que d'essence de thym, **caractérisée en ce qu'**une unité de dosage sous forme de comprimés comprend
| | |
|---|---|
| 2 à 10 mg | d'essence de feuilles du cannelier (90% |
| | d'eugénol) |
| 1 à 20 mg | d'essence de gingembre (20% à 30% de gingérols) |
| 0,5 à 7 mg | d'essence de curcuma (95% de curcumine) |
| 3 à 10 mg | d'huile d'olive |
| 20 à 200 mg | d'acide silique pyrogéné |
toutes les indications pondérales se rapportant à un comprimé de 1000 à 1500 mg.

3. Composition pharmaceutique destinée à l'administration orale et à l'utilisation dans le cadre du traitement de l'excès pondéral chez l'homme ou chez les mammifères, la composition pharmaceutique comprenant un ou plusieurs vanilloïdes, des sels pharmaceutiquement compatibles de ceux-ci en tant que composant a) et un véhicule pharmaceutiquement compatible en tant que composant b), le composant a) étant choisi parmi un groupe constitué d'eugénol, de gingérol, de curcumine et de carvacrol, **caractérisée en ce que** le composant b) est de l'acide silique pyrogéné.

4. Composition pharmaceutique suivant la revendication 3, **caractérisée en ce qu'**une unité de dosage sous forme de comprimé comprend
| | |
|---|---|
| 2 à 20 mg | d'essence de feuilles du cannelier (90% d'eugénol |
| 1 à 20 mg | d'essence de gingembre (20% à 30% de gingérols) |
| 0,5 à 7 mg | d'essence de curcuma (95% de curcumine) |
| 3 à 10 mg | d'huile d'olive |
| 20 à 200 mg | d'acide silique pyrogéné |
toutes les indications pondérales se rapportant à un comprimé de 1000 - 1500 mg.

5. Composition pharmaceutique destinée à l'administration orale et à l'utilisation dans le cadre du traitement de l'excès pondéral chez l'homme ou chez les mammifères, la composition pharmaceutique comprenant une ou plusieurs huiles essentielles, qui contiennent un ou plusieurs vanilloïdes, du carvacrol et/ou des sels pharmaceutiquement compatibles de ceux-ci en tant que composant a) et un véhicule pharmaceutiquement compatible en tant que composant b), le composant a) étant choisi parmi le groupe constitué d'essence de feuilles du cannellier, d'extrait, d'oléorésine ou d'essence de gingembre, d'huile d'extrait ou d'oléorésine de curcuma, ainsi que d'essence de thym, **caractérisée en ce que** le composant b) comprend de l'acide silique pyrogéné.

6. Composition pharmaceutique suivant la revendication 5, **caractérisée en ce qu'**une unité de dosage sous forme de comprimés comprend
| | |
|---|---|
| 2 à 10 mg | d'essence de feuilles du cannelier (90% d'eugénol) |
| 1 à 20 mg | d'essence de gingembre (20% à 30% de gingérols) |
| 0,5 à 7 mg | d'essence de curcuma (95% de curcumine) |
| 3 à 10 mg | d'huile d'olive |
| 20 à 200 mg | d'acide silique pyrogéné |
toutes les indications pondérales se rapportant à un comprimé de 1000 - 1500 mg.

7. Composition pharmaceutique suivant un des préambules des revendications 1 à 3 ou 5, **caractérisée en ce qu'**une unité de dosage sous forme de sirop comprend
| | |
|---|---|
| 2,0 à 7,0 % | d'essence de curcuma (95% de curcumine) |
| 2,0 à 5,0 % | d'essence de feuilles du cannelier (90% d'eugénol) |
toutes les indications pondérales se rapportant à des parts dans le sirop.

8. Composition pharmaceutique suivant une des revendications 2, 5 ou 6 ou suivant la revendication 7, dans la mesure où celle-ci est dépendante des revendications 2 ou 5, **caractérisée en ce que** l'huile essentielle présente une concentration élevée, de préférence >30% en poids des composés appartenant à la classe des vanilloïdes.

9. Composition pharmaceutique suivant une des revendications 3 à 6 ou suivant la revendication 7, dans la mesure où celle-ci est dépendante des revendications 3 ou 5, **caractérisée en ce que** le véhicule comprend au moins une huile végétale, notamment de l'huile d'olive.

10. Composition pharmaceutique suivant une des revendications 1 à 9 ou suivant un des préambules des revendications 1 à 3 ou 5, **caractérisée en ce que** la composition pharmaceutique comprend comme autre composant de l'extrait de cacao, de préférence avec une teneur en théobromine de 6 à 8%.

11. Composition pharmaceutique suivant une des revendications 1 à 10 ou suivant un des préambules des revendications 1 à 3 ou 5, **caractérisée en ce que** la composition pharmaceutique comprend comme autre composant une ou plusieurs proanthocyanidines.

12. Composition pharmaceutique suivant une des revendications 1 à 11 ou suivant un des préambules des revendications 1 à 3 ou 5, **caractérisée en ce que** la composition pharmaceutique comprend comme autre composant une ou plusieurs catéchines.

13. Composition pharmaceutique suivant une des revendications 1 à 12 ou suivant un des préambules des revendications 1 à 3 ou 5, **caractérisée en ce que** la composition pharmaceutique comprend comme autre composant de la phényléthanolamine.

14. Composition pharmaceutique suivant une des revendications 1 à 13 ou suivant un des préambules des revendications 1 à 3 ou 5, **caractérisée en ce que** la composition pharmaceutique comprend comme autre composant de l'acide citrique, au moins une huile essentielle d'agrumes et/ou au moins un extrait d'agrumes.

15. Composition pharmaceutique suivant une des revendications 1 à 14 ou suivant un des préambules des revendications 1 à 3 ou 5, **caractérisée en ce que** la composition chimique comprend comme autre composant une substance ou un mélange de substances avec un effet d'inhibition de l'alpha-amylase, notamment une fraction protéique issue du haricot blanc, du blé ou de la pomme de terre.

16. Composition pharmaceutique suivant la revendication 1, 2, 4 ou 6, **caractérisée en ce que** l'unité de dosage sous forme de comprimé comprend comme autre composant
| | |
|---|---|
| 50 à 100 mg | d'extrait de cacao (6% de théobromine) |
| et ou | |
| 1 à 5 mg | d'acide citrique ou d'huile d'agrumes |
| et ou | |
| 300 à 1000 mg | de fraction protéique du haricot blanc ou du blé |
toutes les indications pondérales se rapportant à un comprimé de 1000 à 2000 mg.

17. Composition pharmaceutique suivant la revendication 7, **caractérisée en ce que** l'unité de dosage sous forme de sirop comprend comme autre composant de l'eau distillée.

18. Composition pharmaceutique suivant la revendication 7 ou 17, **caractérisée en ce que** l'unité de dosage sous forme de sirop comprend comme autre composant :
| | |
|---|---|
| 0,2 à 1,0% | d'huile d'olive |
| et ou | |
| 1,0 à 10,0% | d'extrait de cacao (6% de théobromine) |
| et ou | |
| 5,0 à 25,0% | de fraction protéique du haricot blanc |
| et/ou | |
| 40,0 à 70,0% | eau distillée |
toutes les indications en pour-cent se rapportant à des parts dans le sirop.

19. Utilisation d'une composition pharmaceutique suivant une des revendications 1 à 18 pour la fabrication d'un médicament et/ou d'un complément alimentaire destiné au traitement de l'excès pondéral chez l'homme ou les mammifères.

20. Procédé de fabrication d'une composition pharmaceutique sous forme de comprimé suivant une des revendications 1 à 6 ou 8 à 16 comprenant les étapes suivantes :
i) Mélangeage d'un ou de plusieurs vanilloïdes, de carvacrol et/ou de sels pharmaceutiquement compatibles de ceux-ci avec une huile végétale,
ii) Incorporation de gouttelettes du mélange obtenu à l'étape i) dans de l'acide silique pyrogéné.

21. Procédé de fabrication d'une composition pharmaceutique sous forme de comprimé suivant une des revendications 1 à 6 ou 8 à 16 comprenant les étapes suivantes :
i) Mélangeage d'une ou de plusieurs huiles essentielles, qui contiennent un ou plusieurs vanilloïdes, du carvacrol et/ou des sels pharmaceutiquement compatibles de ceux-ci, avec une huile végétale,
ii) Incorporation de gouttelettes du mélange obtenu au cours de l'étape i) dans de l'acide silique pyrogéné.

22. Procédé suivant la revendication 20 ou 21, **caractérisé en ce qu'**à l'étape ii), on utilise de l'acide silique pyrogéné hautement purifié et non traité.

23. Procédé suivant une des revendications 20 à 22, **caractérisé par** l'étape
iii) Mélangeage du produit obtenu à l'étape ii) à partir d'un vanilloïde, de carvacrol et/ou d'un sel pharmaceutiquement compatible de ceux-ci, c'est-à-dire, d'une huile essentielle, qui contient un ou plusieurs vanilloïdes, du carvacrol et/ou des sels pharmaceutiquement compatibles de ceux-ci, avec au moins un autre produit obtenu à l'étape ii) à partir d'un vanilloïde différent, de carvacrol et/ou d'un sel pharmaceutiquement compatible de ceux-ci, c'est-à-dire, d'une huile essentielle différente, qui contient un ou plusieurs vanilloïdes, du carvacrol et/ou des sels pharmaceutiquement compatibles de ceux-ci.

24. Procédé suivant une des revendications 20 à 23, **caractérisé par** une autre étape, au cours de laquelle au moins une substance porteuse supplémentaire est ajoutée en mélangeant au produit obtenu à l'étape ii) ou iii), substance porteuse, qui est choisie parmi le groupe comprenant la méthylcellulose, la cellulose cristalline 101, la propylméthylcellulose.

25. Procédé de fabrication d'une composition pharmaceutique sous forme de sirop suivant une des revendications 7, 17 ou 18 comprenant l'étape suivante :
j) Mélangeage d'un ou de plusieurs vanilloïdes, de carvacrol et /ou de sels pharmaceutiquement compatibles de ceux-ci avec une huile végétale.

26. Procédé de fabrication d'une composition pharmaceutique sous forme de sirop suivant une des revendications 7, 17 ou 18 comprenant l'étape suivante :
j) Mélangeage d'une ou de plusieurs huiles essentielles comprenant un ou plusieurs vanilloïdes, du carvacrol et/ou des sels pharmaceutiquement compatibles de ceux-ci avec une huile végétale.

27. Procédé suivant la revendication 25 ou 26, **caractérisé par** l'étape
k) Mélangeage du produit obtenu à l'étape j) à partir d'un vanilloïde, de carvacrol et/ou d'un sel pharmaceutiquement compatible de ceux-ci, c'est-à-dire d'une huile essentielle, qui contient un ou plusieurs vanilloïdes, du carvacrol et/ou des sels pharmaceutiquement compatibles de ceux-ci, avec au moins un autre produit obtenu à l'étape j) à partir d'un vanilloïde différent, de carvacrol et ou d'un sel pharmaceutiquement compatible de ceux-ci, c'est-à-dire d'une huile essentielle différente, qui contient un ou plusieurs vanilloïdes, du carvacrol et/ou des sels pharmaceutiquement compatibles de ceux-ci.

28. Procédé suivant une des revendications 20 à 27, **caractérisé par** une autre étape, au cours de laquelle de l'acide citrique, au moins une huile essentielle d'agrumes et/ou au moins un extrait d'agrumes sont ajoutés en mélangeant au produit obtenu à l'étape ii), iii), j) ou k).

29. Procédé suivant une des revendications 20 à 28, **caractérisé par** une autre étape, au cours de laquelle de l'extrait de cacao, de préférence avec une teneur en théobromine de 6 à 8% est ajouté en mélangeant au produit obtenu à l'étape ii ), iii), j) ou k).

30. Procédé suivant une des revendications 20 à 29, **caractérisé par** une autre étape, au cours de laquelle une ou plusieurs catéchines sont ajoutées en mélangeant au produit obtenu à l'étape ii), iii), j) ou k).

31. Procédé suivant une des revendications 20 à 30, **caractérisé par** une autre étape, au cours de laquelle une ou plusieurs proanthocyanidines sont ajoutées en mélangeant au produit obtenu à l'étape ii), iii) ou k).

32. Procédé suivant une des revendications 20 à 30, **caractérisé par** une autre étape, au cours de laquelle de la phényléthanolamine est ajoutée en mélangeant au produit obtenu à l'étape ii), iii), j) ou k).

33. Procédé suivant une des revendications 20 à 32, **caractérisé par** une autre étape, au cours de laquelle une substance ou un mélange de substances avec un effet d'inhibition de l'alpha-amylase, notamment une fraction protéique issue du haricot blanc, du blé ou de la pomme de terre est ajouté en mélangeant au produit obtenu à l'étape ii), iii), j) ou k).
